# EUROPEAN PATENT SPECIFICATION

(11) **EP 3 831 931 B1**
(45) Date of publication and mention of the grant of the patent: **09.04.2025**
(21) Application number: 19845166.8
(22) Date of filing: 02.08.2019
(51) Int. Cl.: C12N 5/00, C12N 5/071, C12N 5/073

(54) **CELL PRODUCTION METHOD**
ZELLPRODUKTIONSVERFAHREN
PROCÉDÉ DE PRODUCTION DE CELLULES

(30) Priority: 03.08.2018 JP 2018146650
(43) Date of publication of application: 09.06.2021
(73) Proprietor: Orizuru Therapeutics, Inc., Fujisawa-shi Kanagawa 251-8555 (JP)
(72) Inventor: YAMAZOE, Noriko, Fujisawa-shi, Kanagawa 251-0012 (JP); HIYOSHI, Hideyuki, Fujisawa-shi, Kanagawa 251-0012 (JP); TOYODA, Taro, Kyoto-shi, Kyoto 606-8501 (JP)
(74) Representative: Jones, Nicholas Andrew
(86) International application number: PCT/JP2019/030437
(87) International publication number: WO 2020/027316

(56) References cited:
- EP-A1- 3 854 869
- JP-A- 2013 536 686
- JP-A- 2016 506 736
- US-A1- 2007 254 359
- US-B2- 10 000 740
- SEKINE K ET AL: "Highly Efficient Generation of Definitive Endoderm Lineage from Human Induced Pluripotent Stem Cells", TRANSPLANTATION PROCEEDINGS, vol. 44, no. 4, 1 March 2012 (2012-03-01), pages 1127 - 1129, XP028482435, ISSN: 0041-1345, [retrieved on 20120317], DOI: 10.1016/J.TRANSPROCEED.2012.03.001
- SEKINE, K. ET AL.: "Highly Efficient Generation of Definitive Endoderm Lineage from Human Induced Pluripotent Stem Cells", TRANSPLANTATION PROCEEDINGS, vol. 44, 2012, pages 1127 - 1129, XP028482435, DOI: 10.1016/j.transproceed.2012.03.001
- QU, SU ET AL.: "Generation of enhanced definitive endoderm from human embryonic stem cells under an albumin/insulin-free and chemically defined condition", LIFE SCIENCES, vol. 175, 2017, pages 37 - 46, XP029977292, DOI: 10.1016/j.lfs.2017.03.017

## Description

### Technical Field

The present invention relates to a method for inducing the differentiation of pluripotent stem cells, which enables definitive endoderm cells or insulin-producing cells to be efficiently induced and/or manufactured from pluripotent stem cells.

### [Background Art]

Research is underway to induce the differentiation of pluripotent stem cells such as induced pluripotent cells or embryonic-stem cells (ES cells) into insulin-secreting cells such as insulin-producing cells or pancreatic β cells and to apply the obtained cells to the treatment of diabetes mellitus. It is known that cells having different features depending on the stages of differentiation appear by inducing the differentiation of pluripotent stem cells (WO2009/012428 and WO2016/021734). For example, the stages of differentiation can be broadly classified into pluripotent stem cells, definitive endoderm cells, primitive gut tube cells, posterior foregut cells, pancreatic progenitor cells, endocrine progenitor cells, insulin-producing cells, and pancreatic β cells in order from relatively undifferentiated to differentiated forms.

Methods for inducing and/or manufacturing cells at each stage of differentiation have previously been researched and reported. For example, Non Patent Literature 1 describes the induction of differentiation of pluripotent stem cells into definitive endoderm cells and states that as a result of examining the influence of addition of a serum replacement B-27 supplement or B-27 supplement (INS(-)) and addition of a phosphoinositide 3-kinase (PI3K) inhibitor LY294002 to a culture medium, the definitive endoderm cells were able to be most effectively induced by the addition of the B-27 supplement (INS(-)).

Non Patent Literature 2 describes the induction of differentiation of pluripotent stem cells into definitive endoderm cells and states that markers for the definitive endoderm cells were highly expressed by culture for 1 day using a culture medium containing 20 µM LY294002 and then culture for 2 days using a culture medium containing 10 µM LY294002.

Non Patent Literature 3 describes a method for producing pancreatic progenitor cells from ES cells and states that a culture medium supplemented with insulin-transferrin-selenium was used for the induction of differentiation of ES cells into definitive endoderm cells in the method.

However, definitive endoderm cells produced by the conventional methods do not sufficiently have a large number of cells or high purity thereof. In the art, there has still been a strong demand for methods for efficiently inducing and/or manufacturing definitive endoderm cells or insulin-producing cells from pluripotent stem cells.

### Citation List

### Patent Literature

Patent Literature 1: WO2009/012428
Patent Literature 2: WO2016/021734

### Non Patent Literature

Non Patent Literature 1: Transplantation Proceedings, 44, 1127-1129 (2012)
Non Patent Literature 2: Nat Commun. 2015 May 22;6: 7212
Non Patent Literature 3: PLoS ONE May 2012, Volume 7, Issue 5, e37004

### Summary of Invention

### Technical Problem

An object of the present invention is to provide a novel approach that enables definitive endoderm cells or insulin-producing cells to be efficiently induced and/or manufactured from pluripotent stem cells.

### Solution to Problem

The present inventors have found that when insulin is allowed to act continuously in inducing the differentiation of pluripotent stem cells into definitive endoderm cells, the obtained cells include many cells other than definitive endoderm cells. On the other hand, the present inventors have found that without the action of insulin in inducing the differentiation of pluripotent stem cells into definitive endoderm cells, a sufficient number of definitive endoderm cells cannot be obtained because the number of cells is not increased.

The present inventors have conducted diligent studies to solve these problems and consequently found that in inducing the differentiation of pluripotent stem cells into definitive endoderm cells, first culture is performed in a differentiation-inducing medium in which insulin acts, and subsequently, second culture is performed in a differentiation-inducing medium in which insulin does not act, whereby definitive endoderm cells can be produced with high purity and the number of cells can be increased, as compared with the case where insulin is allowed to act continuously or is not allowed to act. The present inventors have also found that further differentiated cells can be produced with high purity from the obtained definitive endoderm cells.

The present invention is based on these novel findings and encompasses the following inventions.
[1] A method for producing definitive endoderm cells from pluripotent stem cells, comprising subjecting pluripotent stem cells to first culture in a differentiation-inducing medium in which insulin acts and subsequently to second culture in a differentiation-inducing medium in which insulin does not act.
[2] The method according to [1], wherein
   the first culture is performed in a differentiation-inducing medium comprising insulin, and
   the second culture is performed in a differentiation-inducing medium comprising no insulin.
[3] The method according to [1], wherein
   the first culture is performed in a differentiation-inducing medium comprising insulin and comprising no insulin signaling inhibitor, and
   the second culture is performed in a differentiation-inducing medium comprising insulin and an insulin signaling inhibitor.
[4] The method according to any of [1] to [3], wherein the differentiation-inducing media in which the first culture and the second culture are performed further comprise pyruvate.
[5] The method according to any of [1] to [4], wherein the differentiation-inducing media in which the first culture and the second culture are performed further comprise L-alanyl L-glutamine.
[6] The method according to any of [1] to [5], wherein the differentiation-inducing media in which the first culture and the second culture are performed further comprise 15 mM or more glucose.
[7] The method according to any of [1] to [6], comprising performing the first culture for 6 hours to 48 hours.
[8] The method according to any of [1] to [7], comprising performing the second culture for at least 6 hours.
[9] The method according to any of [1] to [8], wherein the method is carried out in a three-dimensional culture system.
[10] The method according to any of [1] to [8], wherein the method is carried out in a two-dimensional culture system.
[10A] The method according to [9], wherein the pluripotent stem cells are included at 10,000 to 1,000,000 cells/mL at the start of the first culture.
[10B] The method according to [9], wherein the pluripotent stem cells are included at 100,000 to 500,000 cells/mL at the start of the first culture.
[10C] The method according to [10], wherein the pluripotent stem cells are included at 50,000 to 1,000,000 cells/cm² at the start of the first culture.
[11] The method according to [10], wherein the pluripotent stem cells are included at 150,000 to 300,000 cells/cm² at the start of the first culture.
[12] The method according to any of [1] to [11], wherein the differentiation-inducing medium is based on DMEM (Dulbecco's modified Eagle medium).
[13] The method according to any of [1] to [12], wherein the differentiation-inducing medium for the first culture comprises a ROCK inhibitor and/or a GSK3β inhibitor.
[14] A method for producing insulin-producing cells, comprising the step of further inducing the differentiation of definitive endoderm cells produced by subjecting pluripotent stem cells to first culture in a differentiation-inducing medium in which insulin acts and subsequently to second culture in a differentiation-inducing medium in which insulin does not act.
[15] A method for producing insulin-producing cells, comprising the step of further inducing the differentiation of definitive endoderm cells produced by subjecting pluripotent stem cells to first culture in a differentiation-inducing medium comprising insulin and subsequently to second culture in a differentiation-inducing medium comprising no insulin.
[16] A method for producing insulin-producing cells, comprising the step of further inducing the differentiation of definitive endoderm cells produced by subjecting pluripotent stem cells to first culture in a differentiation-inducing medium comprising insulin and comprising no insulin signaling inhibitor and subsequently to second culture in a differentiation-inducing medium comprising insulin and an insulin signaling inhibitor.
[17] Definitive endoderm cells produced by a method according to [1].
[18] Insulin-producing cells produced by a method according to any of [14] to [16].
[19] A medicament comprising insulin-producing cells according to [18].
[20] The method according to any of [1] to [13], wherein the definitive endoderm cells are produced with the rate of coexisting SOX2-positive (SOX2+) cells being less than 5%.
[21] The method according to any of [1] to [13] and [20], wherein the number of produced definitive endoderm cells is twice or more the number of initially seeded pluripotent stem cells.
[22] A method for producing posterior foregut cells, comprising the step of inducing the differentiation of definitive endoderm cells produced by a method according to any of [1] to [13], [20], and [21] into posterior foregut cells.
[23] The method according to [22], wherein the produced posterior foregut cells comprise 90% or more of PDX1-positive (PDX1+) cells.
[24] The method according to [22] or [23], wherein the number of produced posterior foregut cells is twice or more the number of initially seeded pluripotent stem cells.
[25] A method for producing pancreatic progenitor cells, comprising the step of inducing the differentiation of definitive endoderm cells produced by a method according to any of [1] to [13], [20], and [21] into pancreatic progenitor cells.
[26] The method according to [25], wherein the produced pancreatic progenitor cells comprise 80% or more of PDX1-positive (PDX1+) and NKX6.1-positive (NKX6.1+) cells.
[27] The method according to [25] or [26], wherein the number of produced pancreatic progenitor cells is twice or more the number of initially seeded pluripotent stem cells.
[28] Definitive endoderm cells with the rate of coexisting SOX2-positive (SOX2+) cells being less than 5%, the definitive endoderm cells being produced by a method according to any of [1] to [13], [20], and [21].
[29] Posterior foregut cells comprising 90% or more of PDX1-positive (PDX1+) cells, the posterior foregut cells being produced by a method according to any of [22] to [24].
[30] Pancreatic progenitor cells comprising 80% or more of PDX1-positive (PDX1+) and NKX6.1-positive (NKX6.1+) cells, the pancreatic progenitor cells being produced by a method according to any of [25] to [27].

The present specification encompasses the contents described in the specification and/or drawings of Japanese Patent Application No. 2018-146650 on which the priority of the present application is based.

### Advantageous Effects of Invention

The present invention can provide a novel approach that enables definitive endoderm cells or insulin-producing cells to be efficiently induced and/or manufactured from pluripotent stem cells.

### Brief Description of Drawings

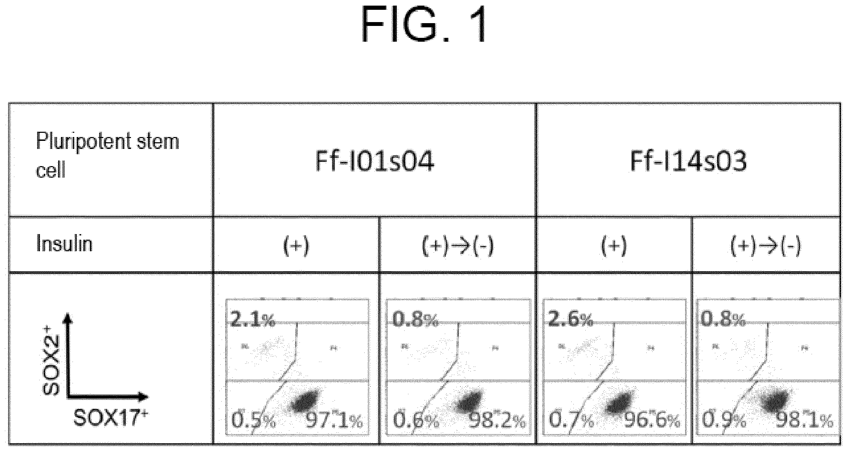

[Figure 1] Figure 1 shows results of analyzing the percentages of SOX2-positive/negative and SOX17-positive/negative cells in obtained cells using a flow cytometer in the induction of differentiation of iPS cells (Ff-I01s04 line and Ff-I14s03 line) into definitive endoderm cells, when the iPS cells were first cultured in a differentiation-inducing medium containing insulin and subsequently cultured in a differentiation-inducing medium containing no insulin (insulin(+) → (-)) or when the iPS cells were cultured only in a differentiation-inducing medium containing insulin (insulin(+)).

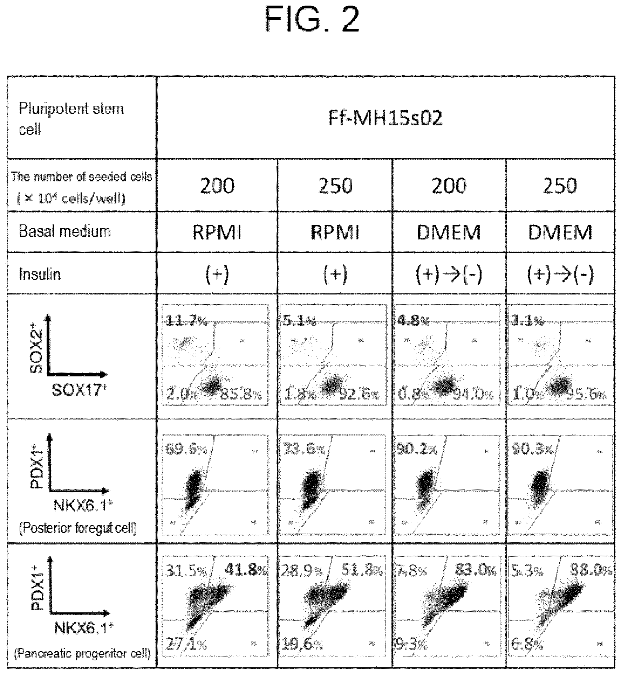

[Figure 2] Figure 2 shows results of analyzing the percentages of SOX2-positive/negative and SOX17-positive/negative cells in obtained cells using a flow cytometer in the induction of differentiation of iPS cells (Ff-MH15s02 line) into definitive endoderm cells, where RPMI medium or DMEM medium was used as a basal medium for a differentiation-inducing medium, when, in the case of using RPMI medium as a base medium, the iPS cells were cultured from day 0 through day 3 in a differentiation-inducing medium containing insulin, or when, in the case of using DMEM medium as a base medium, the iPS cells were first (day 0) cultured in a differentiation-inducing medium containing insulin and subsequently (day 1 to day 3) cultured in a differentiation-inducing medium containing no insulin. Figure 2 further shows results of analyzing the percentages of PDX1-positive/negative and NKX6.1-positive/negative cells in cells (posterior foregut cells and pancreatic progenitor cells) obtained by further inducing the differentiation of the cells obtained by each approach, using a flow cytometer.

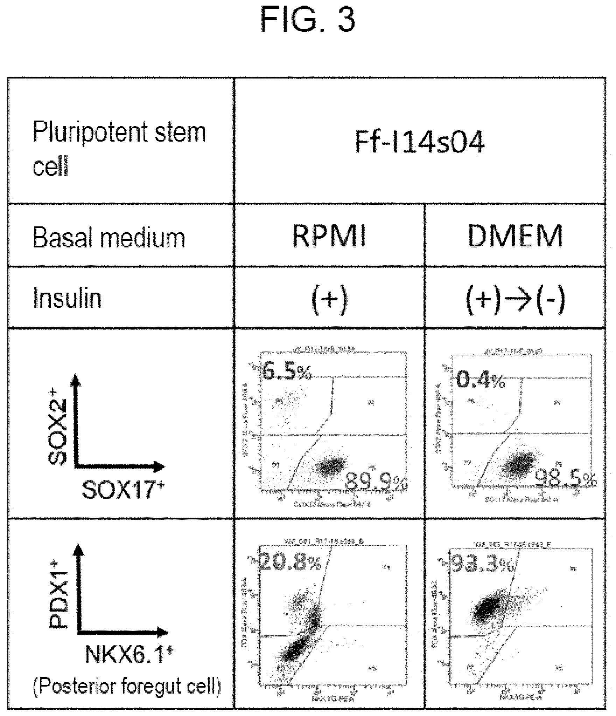

[Figure 3] Figure 3 shows results of analyzing the percentages of SOX2-positive/negative and SOX17-positive/negative cells in obtained cell masses using a flow cytometer in the induction of differentiation of iPS cells (Ff-I14s04 line) into definitive endoderm cells, when the iPS cells were first three-dimensionally cultured in a differentiation-inducing medium containing insulin and subsequently three-dimensionally cultured in a differentiation-inducing medium containing no insulin (insulin(+) → (-)) or when the iPS cells were three-dimensionally cultured only in a differentiation-inducing medium containing insulin (insulin(+)). Figure 3 further shows results of analyzing the percentages of PDX1-positive/negative and NKX6.1-positive/negative cells in cell masses (posterior foregut cells) obtained by further three-dimensionally culturing the cell mass obtained by each approach and thereby inducing their differentiation, using a flow cytometer.

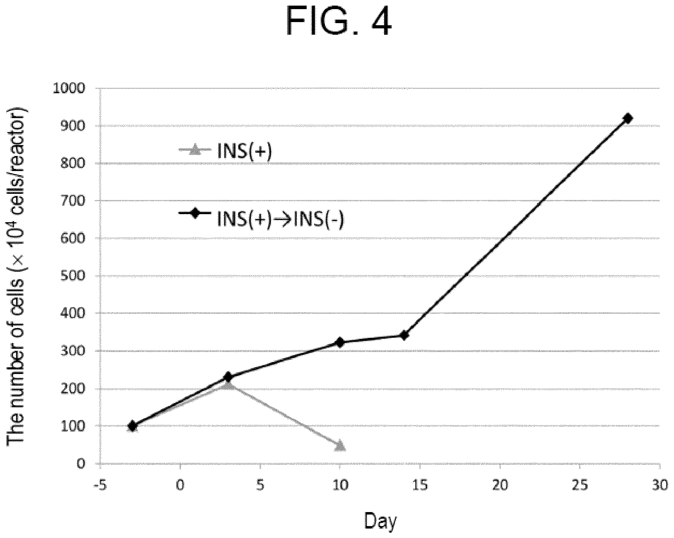

[Figure 4] Figure 4 is a graph showing time-dependent change in the number of cells in each cell mass in the induction of differentiation of iPS cells (Ff-I14s04 line) into definitive endoderm cells, when the iPS cells were first three-dimensionally cultured in a differentiation-inducing medium containing insulin and subsequently three-dimensionally cultured in a differentiation-inducing medium containing no insulin to obtain a cell mass (INS(+) → INS(-)), or the iPS cells were three-dimensionally cultured only in a differentiation-inducing medium containing insulin to obtain a cell mass (INS(+)), and the obtained cell masses were induced to differentiate by three-dimensional culture.

### Description of Embodiments

### 1. Terminology

Hereinafter, the terms described herein will be described.

As used herein, "about" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

As used herein, "without the use of feeder cell(s)" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts may be used or a primary culture of murine embryonic fibroblasts or immortalized murine embryonic fibroblasts may be used. The feeder cells can be inactivated by exposure to radiation or treatment with mitomycin C.

As used herein, "adhered (adherent)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating (growing), and/or differentiating cells out of tissue or the living body, for example, in a cell culture dish or flask. The culture includes two-dimensional culture (plane culture) and three-dimensional culture (suspension culture).

As used herein, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

As used herein, "deplete(s)" and "depletion" refer to decreasing the amount of a certain component in cells or a composition such as a composition of cells and "depleted" refers, when used to describe cells or a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

As used herein, "purify(ies)" and "purification" refer to removing impurities in a composition such as a composition of cells and making it pure for a certain component and "purified" refers, when used to describe a composition of cells, for example, a cell population, to a cell population in which the amount of impurities is decreased in comparison with the percentage of such components in the cell population before purification and the purity of a certain component is improved. For example, a composition such as a cell population can be purified for a target cell type and, accordingly, the percentage of the target cell type is increased in comparison with the percentage of the target cells present in the cell population before the purification. A cell population can be purified for a target cell type by a method of selecting and sorting cells known in the art. A cell population can be purified by a specific process of sorting or selection described herein. In a certain embodiment of the present invention, the purity of a target cell population is brought by a method of purifying a target cell population to at least 70%, 80%, 85%, 90%, 95%, 97%, 98%, or 99% or to the extent at which impurities (including contaminant cells) are undetectable.

As used herein, "marker" means a cell antigen or a gene thereof that is specifically expressed depending on a predetermined cell type, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. The detection of a marker gene can be conducted by a method of amplifying and/or detecting nucleic acid known in the art, for example, RT-PCR, microarray, biochip, or the like. As used herein, "positive" for a marker protein means being detected to be positive by flow cytometry and "negative" therefor means being equal to or less than the lower detection limit in flow cytometry. Also, "positive" for a marker gene means being detected by RT-PCR and "negative" therefor means being equal to or less than the lower detection limit in RT-PCR.

As used herein, "expression" is defined as transcription and/or translation of a certain nucleotide sequence driven by an intracellular promoter.

As used herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

As used herein, "multipotency" means the ability to differentiate into plural and limited numbers of linages of cells. For example, mesenchymal stem cells, hematopoietic stem cells, neural stem cells are multipotent, but not pluripotent.

As used herein, "pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, preferably, pluripotent stem cells are human pluripotent stem cells.

Available "ES cells" include murine ES cells, such as various murine ES cell lines established by inGenious, RIKEN, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2, the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, RWMH15s02, Ff-MH15s02, 253G1, 201B7, 409B2, 454E2, 606A1, 610B1, and 648A1, and the CDI cell lines MyCell iPS Cells (21525.102.10A), MyCell iPS Cells (21526.101.10A), and the like.

"Definitive endoderm cells" mean cells characterized by the expression of at least one of the markers SOX17, FOXA2, BMP2, CER, and CXCR4.

"Primitive gut tube cells" mean cells characterized by the expression of at least one of the markers HNF1B and HNF4A.

"Posterior foregut cells" mean cells characterized by the expression of at least one of the markers PDX-1, HNF6, and HLXB9.

"Pancreatic progenitor cells" mean cells characterized by the expression of at least one of the markers PDX-1, NKX6.1, PTF-1α, GATA4 and SOX9.

"Endocrine progenitor cells" mean cells characterized by the expression of at least one of the markers Chromogranin A, NeuroD and NGN3 and no expression of a marker of the pancreas-related hormone system (for example, insulin). The endocrine progenitor cells may express a marker such as PAX-4, NKX2-2, Islet-1, PDX-1, or PTF-1α.

"Insulin-producing cells" mean cells characterized by the expression of insulin. "Insulin-producing cells" can be preferably further characterized by the expression of NKX6.1 in addition to the expression of insulin. Specifically, "Insulin-producing cells" more preferably mean cells that express both markers of insulin and NKX6.1.

"Pancreatic β cells" mean cells more mature than "insulin-producing cells" and specifically means cells characterized by expressing at least one of the markers MAFA, UCN3, and IAPP, which are maturation markers of pancreatic β cells, or by a reaction to increase insulin secretion by glucose stimulation.

Cells at each stage of differentiation can be produced by an approach mentioned below in detail.

As used herein, "cells" mean a composition of cells, i.e., a cell population, unless otherwise specified. Thus, "cells" may include not only specific cells but one or more other cells. The percentage of specific cells in "cells" can be elevated by enriching or purifying the specific cells or by depleting one or more other cells.

As used herein, "factor having CDK8/19-inhibiting activity" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other. Usually, the inhibition of CDK8 also involves the inhibition of CDK19.

"Growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony-stimulating factors (for example, granulocyte/macrophage colony-stimulating factor (GM-CSF)), various interferons (for example, IFN-y, and the like), and other cytokines having effects on stem cells, for example, stem cell factor (SCF), and erythropoietin (Epo).

As used herein, "ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be also referred to as Y-27632), Fasudil (HA1077), (2S)-2-methyl-1-[(4-methyl-5-isoquinolinyl]sulfonyl]hexahydro-1H-1,4-diazepine (H-1152), 4β-[(1R)-1-aminoethyl]-N-(4-pyridyl)benzene-1zencarboxamide (Wf-536), N-(1H-pyrrolo[2,3-b]pyridin-4-yl)-4PER(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used as ROCK inhibitors, and commercially available, or synthesized according to a known method.

As used herein, "GSK3β inhibitors" are substances having the inhibitory activity for GSK3β (glycogen synthase kinase 3β). GSK3 (glycogen synthase kinase 3) is a kind of a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β is not limited to these and antisense oligonucleotides and siRNA to GSK3β mRNA, antibodies that bind to GSK3β, dominant negative GSK3β mutants, and the like can also be used as GSK3β inhibitors, and commercially available, or synthesized according to a known method.

As used herein, examples of "serum replacement" include Knockout Serum Replacement (KSR: Invitrogen), StemSure Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure Serum Replacement, ITS-G. The concentration of serum replacement in a medium when added into a medium is 0.01-10% by weight, and preferably 0.1-2% by weight. In the present invention, "serum replacement" is preferably used instead of serum. In the present specification, B-27 supplement containing insulin is also referred to as "B-27 supplement (INS(+))", and B-27 supplement containing no insulin is also referred to as "B-27 supplement (INS(-))".

### 2. Method for producing definitive endoderm cells from pluripotent stem cells

In the present invention, definitive endoderm cells can be produced by subjecting pluripotent stem cells to first culture in a differentiation-inducing medium in which insulin acts and subsequently to second culture in a differentiation-inducing medium in which insulin does not act.

### (1) First culture

"insulin acts" mean conditions that activate an insulin signal transduction pathway in cells by insulin. Usually, insulin binds to an insulin receptor present on cell membrane surface so that tyrosine kinase incorporated in the receptor is activated for the tyrosine phosphorylation of the insulin receptor substrate protein family (IRS: IRS-1,2,3). In the present specification, causing this series of reactions initiated by the binding of insulin to an insulin receptor is referred to as "activating an insulin signal transduction pathway".

Examples of the conditions with action of insulin include the case where the differentiation-inducing medium comprises insulin. The insulin can be insulin that can activate an insulin signal transduction pathway in pluripotent stem cells, and may be produced by a recombination method or may be produced through synthesis by a solid-phase synthesis method. Insulin derived from a human, a nonhuman primate, a pig, cattle, a horse, sheep, a goat, a llama, a dog, a cat, a rabbit, a mouse, a guinea pig, or the like can be used. Human insulin is preferred. In the present specification, insulin is also referred to as "INS". The case of containing insulin is also referred to as "INS(+)", and the case of containing no insulin is also referred to as "INS(-)".

In the present invention, an insulin mutant, an insulin derivative or an insulin agonist may be used as "insulin" as long as it can activate an insulin signal transduction pathway in pluripotent stem cells. Examples of "insulin mutant" include ones having a polypeptide that consists of an amino acid sequence derived from the amino acid sequence of insulin by the deletion, substitution, addition or insertion of 1 to 20, preferably 1 to 10, more preferably 1 to 5 amino acids and is capable of activating an insulin signal transduction pathway, or a polypeptide that consists of an amino acid sequence having 80% or more, preferably 90% or more, more preferably 95% or more, most preferably 99% or more sequence identity to the amino acid sequence of insulin and is capable of activating an insulin signal transduction pathway. Amino acid sequences can be compared by a known approach. The comparison can be carried out using, for example, BLAST (Basic Local Alignment Search Tool at the National Center for Biological Information), for example, at default settings. "Insulin derivative" means a polypeptide that consists of an amino acid sequence obtained by the chemical substitution (for example, α-methylation, α-hydroxylation), deletion (for example, deamination), or modification (for example, N-methylation) of one or some of groups of amino acid residues of insulin or an insulin mutant, and is capable of activating an insulin signal transduction pathway, or a substance having a similar effect. "Insulin agonist" means a polypeptide capable of activating an insulin signal transduction pathway by binding to an insulin receptor, regardless of the structure of insulin, or a substance having a similar effect.

The differentiation-inducing medium for the first culture can comprise the insulin in an amount of 0.01 to 20 µM, preferably 0.1 to 10 µM, more preferably 0.5 to 5 µM. The concentration of the insulin in the differentiation-inducing medium may be the concentration of insulin contained in added B-27 supplement, but is not limited thereto.

The differentiation-inducing medium can further comprise activin A in addition to the insulin. The activin A can be contained in an amount of 5 to 200 ng/mL, preferably 10 to 150 ng/mL, more preferably 30 to 120 ng/mL, particularly preferably about 100 ng/mL, in the medium. In another embodiment, the concentration of the activin A in the medium is about 0.1 to 100 ng/ml, preferably about 1 to 50 ng/ml, more preferably about 3 to 10 ng/ml.

The differentiation-inducing medium can further comprise a ROCK inhibitor and/or a GSK3β inhibitor. The concentration of the ROCK inhibitor in the medium is appropriately set depending on the type of the ROCK inhibitor used. For example, in the case of using Y27632 as the ROCK inhibitor, its concentration can be usually 5 to 20 µM, preferably 5 to 15 µM, particularly preferably about 10 µM. The concentration of the GSK3β inhibitor in the medium is appropriately set depending on the type of the GSK3β inhibitor used. For example, in the case of using CHIR99021 as the GSK3β inhibitor, its concentration can be usually 2 to 5 µM, preferably 2 to 4 µM, particularly preferably about 3 µM.

The differentiation-inducing medium can further comprise one or more members selected from the group consisting of pyruvate (sodium salt, etc.), L-alanyl L-glutamine, and glucose. The pyruvate can be contained in an amount of 10 to 1000 mg/L, preferably 30 to 500 mg/L, more preferably 50 to 200 mg/L, particularly preferably about 110 mg/L, in the medium. The L-alanyl L-glutamine can be contained in an amount of 50 to 2000 mg/L, preferably 100 to 1500 mg/L, more preferably 500 to 1000 mg/L, particularly preferably about 860 mg/L, in the medium. The glucose can be contained in an amount of 15 mM or more, preferably 15 to 30 mM, more preferably 15 to 25 mM, particularly preferably about 25 mM, in the medium. The concentrations of the pyruvate, the L-alanyl L-glutamine and the glucose in the differentiation-inducing medium may be the concentrations of pyruvate, L-alanyl L-glutamine and glucose contained in DMEM medium (DMEM, high glucose, GlutaMAX(TM), pyruvate (Thermo Fisher Scientific)) or other DMEM media, but are not limited thereto.

The differentiation-inducing medium can further comprise dimethyl sulfoxide.

The differentiation-inducing medium is based on a basal medium for use in the culture of mammalian cells and can be supplemented with one or more of the components described above for use. Examples of the basal medium can include RPMI medium, MEM medium, iMEM medium, DMEM (Dulbecco's modified Eagle medium) medium, Improved MEM Zinc Option medium, Improved MEM/1% B-27 supplement/Penicillin Streptomycin medium, and MCDB131/10 mM Glucose/20 mM Glucose/NaHCO₃/FAF-BSA/ITS-X/Glutamax/ascorbic acid/Penicillin Streptomycin medium. DMEM medium is preferred, and DMEM medium containing pyruvate, L-alanyl L-glutamine, and glucose in the amounts described above is more preferred.

The culture period of the first culture can be in a range selected from 6 hours to 48 hours, preferably 12 to 24 hours. The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture. The number of cells at the start of the culture is not particularly limited and can be 50,000 to 1,000,000 cells/cm², preferably 150,000 to 300,000 cells/cm², more preferably about 200,000 cells/cm², for two-dimensional culture. The number of cells at the start of the culture is not particularly limited and can be 10,000 to 1,000,000 cells/mL, preferably 100,000 to 500,000 cells/mL, for three-dimensional culture.

### (2) Second culture

"insulin does not act" mean conditions that do not activate an insulin signal transduction pathway in cells by insulin. "Not activate an insulin signal transduction pathway in cells" not only means that there occurs no activation of the insulin signal transduction pathway but means that there occurs slight activation to an extent that no significant difference is found as compared with the activation of the insulin signal transduction pathway in the absence of insulin. Thus, examples of "insulin does not act" include the absence of insulin in the differentiation-inducing medium, and, even if insulin is contained in the differentiation-inducing medium, conditions where the insulin is contained in an amount that causes the slight activation to an extent that no significant difference is found. Alternatively, "insulin does not act" also mean that, even if insulin is contained in the differentiation-inducing medium, the insulin signal transduction pathway is not activated owing to the coexistence of an insulin signaling inhibitor. "Insulin signaling inhibitor" means a component capable of blocking an insulin signal transduction pathway at any position. Examples of such an insulin signaling inhibitor include polypeptides and compounds that bind to or compete with an insulin, an insulin receptor, or various proteins or the like acting as a signal transducer and thereby inhibit intermolecular interaction involving these factors. Examples of such an insulin signaling inhibitor include LY294002 [2-(4-morpholinyl)-8-phenyl-4H-1-benzopyran-4-one] which competitively inhibits ATP binding to a catalytic subunit of PI3 kinase. The insulin signaling inhibitor is not limited to these, and, for example, an antibody binding to an insulin, an insulin receptor, or any of various proteins acting as a signal transducer, a dominant negative mutant thereof, or an antisense oligonucleotide or siRNA against mRNA of an insulin receptor or any of various proteins acting as a signal transducer can also be used as the insulin signaling inhibitor. The insulin signaling inhibitor is commercially available or can be synthesized according to a known method.

The differentiation-inducing medium for the second culture can comprise activin A. The amount of the activin A in the medium can be selected from the range described about the first culture and may be the same as or different from the amount used for the first culture.

The differentiation-inducing medium can further comprise a ROCK inhibitor and/or a GSK3β inhibitor. The amount of the ROCK inhibitor and/or the GSK3β inhibitor in the medium can be selected from the range described about the first culture and may be the same as or different from the amount used for the first culture.

The differentiation-inducing medium can further comprise one or more members selected from the group consisting of pyruvate, L-alanyl L-glutamine, and glucose. The amounts of the pyruvate, the L-alanyl L-glutamine, and the glucose in the medium can be selected from the ranges described about the first culture and may be the same as or different from the amounts used for the first culture.

The differentiation-inducing medium can further comprise dimethyl sulfoxide.

The differentiation-inducing medium for use in the second culture is based on a basal medium for use in the culture of mammalian cells and can be supplemented with one or more of the components described above for use. The basal medium described about the first culture can be used, and the basal medium may be the same as or different from that used for the first culture. DMEM medium is preferred, and DMEM medium containing pyruvate, L-alanyl L-glutamine, and glucose in the amounts described above is more preferred.

The culture period of the second culture is at least 6 hours and can be in a range selected from preferably 6 to 72 hours, more preferably 24 hours to 72 hours. The culture temperature is not particularly limited, and the culture is performed at 30 to 40°C (for example, 37°C). The culture may be performed by any of two-dimensional culture and three-dimensional culture. The concentration of carbon dioxide in a culture container is on the order of, for example, 5%.

Specifically, the method of the present invention involves culture for 1 day or 2 days in DMEM medium supplemented with insulin, activin A, a ROCK inhibitor, and a GSK3β inhibitor (first culture) and further culture for 2 days or 3 days in DMEM medium supplemented with activin A (second culture).

The method of the present invention can produce definitive endoderm cells with high purity or a large number of cells. Specifically, definitive endoderm cells obtained by the method of the present invention are a cell population comprising definitive endoderm cells with high purity, wherein the rate of coexisting SOX2-positive (SOX2+) cells indicating remaining pluripotent stem cells or cells of other lineages is less than 5%, preferably less than 1%, more preferably less than 0.1%. Furthermore, the number of definitive endoderm cells produced by the method of the present invention is larger than that of initially seeded pluripotent stem cells and is, for example, twice or more the number of initially seeded pluripotent stem cells.

The obtained definitive endoderm cells can be subjected to the step of further inducing differentiation and can be used in the production of primitive gut tube cells, posterior foregut cells, pancreatic progenitor cells, endocrine progenitor cells, or insulin-producing cells.

### 3. Methods for producing cells at various stages of differentiation

The obtained definitive endoderm cells can be induced to differentiate into primitive gut tube cells, posterior foregut cells, pancreatic progenitor cells, endocrine progenitor cells, or insulin-producing cells by use of a known approach. Specifically, cells at various stages of differentiation, such as insulin-producing cells, can be obtained through differentiation into cells at each stage of differentiation by use of the following steps of inducing differentiation:
step 1) inducing the differentiation of the definitive endoderm cells into primitive gut tube cells;
step 2) inducing the differentiation of the primitive gut tube cells into posterior foregut cells;
step 3) inducing the differentiation of the posterior foregut cells into pancreatic progenitor cells;
step 4) inducing the differentiation of the pancreatic progenitor cells into endocrine progenitor cells; and
step 5) inducing the differentiation of the endocrine progenitor cells into insulin-producing cells.

Hereinafter, each step will be described. However, the induction of differentiation of cells at each stage of differentiation is not limited by these approaches.

### Step 1) Differentiation into primitive gut tube cells

The definitive endoderm cells obtained through the second culture are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

The basal medium for use in the culture of mammalian cells described about the first culture can be used as medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

### Step 2) Differentiation into posterior foregut cells

The primitive gut tube cells obtained in step 1) are further cultured in a medium containing a growth factor, cyclopamine, noggin, and the like to induce their differentiation into posterior foregut cells. The culture period is 1 day to 5 days, preferably about 2 days. The culture may be performed by any of two-dimensional culture and three-dimensional culture.

Cyclopamine is replaceable with a SHH inhibitor SANT-1 ((4-benzyl-piperazin-1-yl)-(3,5-dimethyl-1-phenyl-1H-pyrazol-4-ylmethylene)-amine) or jervine ((3β,23β)-17,23-epoxy-3-hydroxyveratraman-11-one).

Noggin, a BMP-4 antagonist, is replaceable with a BMP receptor inhibitor LDN193189 (4-[6-(4-piperazin-1-yl-phenyl)-pyrazolo[1,5-α]pyrimidin-3-y1]-quinoline hydrochloride).

The culture temperature is not particularly limited, and the culture is performed at 30 to 40°C (for example, 37°C). The concentration of carbon dioxide in a culture container is on the order of, for example, 5%.

The basal medium for use in the culture of mammalian cells described about the first culture can be used as medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 µM, preferably 0.3 to 1.0 µM, particularly preferably about 0.5 µM.

The concentration of the noggin in the medium is not particularly limited and is usually 10 to 200 ng/mL, preferably 50 to 150 ng/mL, particularly preferably about 100 ng/mL.

The method of the present invention can produce posterior foregut cells with high purity and a large number of cells. Specifically, posterior foregut cells produced through the produced definitive endoderm cells by the method of the present invention are a cell population comprising posterior foregut cells with high purity, wherein PDX1-positive (PDX1+) cells are contained at a percentage of 90% or more, preferably 95% or more, more preferably 98% or more. Furthermore, the number of posterior foregut cells produced by the method of the present invention is larger than that of initially seeded pluripotent stem cells and is, for example, twice or more the number of initially seeded pluripotent stem cells.

### Step 3) Differentiation into pancreatic progenitor cells

The posterior foregut cells obtained in step 2) are further cultured in a medium containing a factor having CDK8/19-inhibiting activity, preferably a medium containing a factor having CDK8/19-inhibiting activity and a growth factor, to induce their differentiation into pancreatic progenitor cells. The culture period is 2 days to 10 days, preferably about 5 days.

The culture may be performed by any of two-dimensional culture and three-dimensional culture. In the case of two-dimensional culture, according to the previous report (Toyoda et al., Stem cell Research (2015) 14, 185-197), the posterior foregut cells obtained in step 2) are treated and dispersed by pipetting with 0.25% trypsin-EDTA, which is then removed by centrifugal separation, after which the resulting cells are suspended in a fresh medium of step 3) and reseeded.

The basal medium for use in the culture of mammalian cells described about the first culture can be used as medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 µM to 5 µM, preferably 0.00001 µM to 1 µM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF and EGF as the growth factor, the concentration of EGF is usually 5 to 150 ng/mL, preferably 30 to 100 ng/mL, particularly preferably about 50 ng/mL, and the concentration of KGF is usually 10 to 200 ng/mL, preferably 50 to 150 ng/mL, particularly preferably about 100 ng/mL.

Culture on the first day in step 3) may be performed in the presence of a ROCK inhibitor, and culture on the following days may be performed in a medium containing no ROCK inhibitor.

The medium may also contain a PKC activator. PdBU (PKC activator II), TPB (PKC activator V), or the like is used as the PKC activator, though the PKC activator is not limited thereto. The concentration of the PKC activator to be added is about 0.1 to 100 ng/ml, preferably about 1 to 50 ng/ml, more preferably about 3 to 10 ng/ml. The medium may also be supplemented with dimethyl sulfoxide and/or activin (1 to 50 ng/ml).

In any of the steps, the medium may be supplemented with a serum replacement, in addition to the components described above. Also, an amino acid, L-glutamine, GlutaMAX (product name), a non-essential amino acid, a vitamin, nicotinamide, an antibiotic (for example, Antibiotic-Antimycotic (also referred to as AA herein), penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight.

The cell culture may be performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

The pancreatic progenitor cells obtained in step 3) can be further purified using a known surface marker glycoprotein 2 (GP2) or the like. The purification can be performed by a method known per se, for example, using anti-GP2 antibody-immobilized beads.

The method of the present invention can produce pancreatic progenitor cells with high purity and a large number of cells. Specifically, pancreatic progenitor cells produced through the produced definitive endoderm cells by the method of the present invention are a cell population comprising pancreatic progenitor cells with high purity, wherein PDX1-positive (PDX1+) and NKX6.1-positive (NKX6.1+) cells are contained at a percentage of 80% or more, preferably 85% or more, more preferably 90% or more. Furthermore, the number of pancreatic progenitor cells produced by the method of the present invention is larger than that of initially seeded pluripotent stem cells and is, for example, twice or more the number of initially seeded pluripotent stem cells.

### Step 4) Differentiation into endocrine progenitor cells

The pancreatic progenitor cells obtained in step 3) are further cultured in a medium containing a growth factor to induce their differentiation into endocrine progenitor cells. The culture may be performed by any of two-dimensional culture and three-dimensional culture. In the case of two-dimensional culture, the pancreatic progenitor cells obtained in step 3) are treated and dispersed by pipetting with 0.25% trypsin-EDTA, which is then removed by centrifugal separation, after which the resulting cells are suspended in a fresh medium of step 4) and reseeded. The culture period is 2 days to 3 days, preferably about 2 days.

The basal medium for use in the culture of mammalian cells described about the first culture can be used as medium. The medium is supplemented with SANT1, retinoic acid, ALK5 inhibitor II, T3, and LDN according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. The medium may also be supplemented with dimethyl sulfoxide.

The cell culture may be performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

### Step 5) Differentiation into insulin-producing cells

The endocrine progenitor cells obtained in step 4) are further cultured in a medium containing a growth factor to induce their differentiation into insulin-producing cells. The culture period is 10 days to 30 days, preferably about 10 to 20 days.

The basal medium for use in the culture of mammalian cells described about the first culture can be used as medium. The medium is supplemented with ALK5 inhibitor II, T3, LDN, γ-secretase inhibitor XX, γ-secretase inhibitor RO, N-cysteine, an AXL inhibitor, and ascorbic acid according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428.

The culture may be performed by any of two-dimensional culture and three-dimensional culture. The cell culture may be performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

### 4. Differentiation into pancreatic β cells

The insulin-producing cells obtained in the preceding step can be induced to differentiate into pancreatic β cells by transplantation into a living body of an animal.

"Animal" is preferably a mammal. Examples thereof include humans, nonhuman primates, pigs, cattle, horses, sheep, goats, llamas, dogs, cats, rabbits, mice, and guinea pigs. A human is preferred.

The transplantation is preferably performed to an *in vivo* region where the cell can be fixed at a given position, and can be performed, for example, subcutaneously, intraperitoneally, to the peritoneal mesothelium, to the greater omentum, to a fat tissue, to a muscle tissue, or beneath the capsule of each organ such as the pancreas or the kidney, in the animal. The number of cells to be transplanted may vary depending on factors such as the stage of differentiation of the cells to be transplanted, the age and body weight of a recipient, the size of a transplantation site, and the severity of a disease and is not particularly limited. For example, the number of cells can be on the order of 10 × 10⁴ cells to 10 × 10¹¹ cells. The transplanted cells are induced to differentiate in an *in vivo* environment and can thereby differentiate into the cells of interest, preferably pancreatic β cells. The obtained pancreatic β cells may then be recovered or may be indwelled *in vivo* as they are.

The insulin-producing cells or the pancreatic β cells obtained by the approach described above are transplanted as they are or in a capsule form to an affected area and are thereby useful as a cell medicine for treating diabetes mellitus, particularly, type I diabetes mellitus.

The insulin-producing cells or the pancreatic β cells may be a prodrug. The prodrug refers to cells that are differentiated after transplantation into a living body and converted to cells having a function of treating a disease.

The insulin-producing cells or the pancreatic β cells obtained by the approach described above have low toxicity (for example, acute toxicity, chronic toxicity, genetic toxicity, reproductive toxicity, cardiotoxicity, and carcinogenicity) and can be safely administered as they are or in the form of a pharmaceutical composition containing the cells mixed with a pharmacologically acceptable carrier, etc. to a mammal (for example, a mouse, a rat, a hamster, a rabbit, a cat, a dog, cattle, sheep, a monkey, and a human).

Hereinafter, the present invention will be described with reference to Examples. However, the present invention is not limited by these Examples.

### Examples

The induction of differentiation of pluripotent stem cells into definitive endoderm cells, posterior foregut cells, pancreatic progenitor cells, and the like was carried out by the first culture and the second culture described above and in accordance with methods described in the previous report (Stem Cell Research (2015) 14, 185-197), etc.

The amount of B-27 supplement (INS(+)) or B-27 supplement (INS(-)) (Thermo Fisher Scientific) added was set such that the final concentration was 2% according to manufacturer's instruction (days 0 to 3) (the final concentration was set to 1% on day 4 or later).

### [I] Induction of differentiation into definitive endoderm cells (two-dimensional culture) involving change from INS(+) medium to INS(-) medium

### (1) Method

### (i) Cells

An induced pluripotent cell line Ff-I01s04 or Ff-I14s03 was used as pluripotent stem cells.

### (ii) Differentiation-inducing medium and culture schedule

B-27 supplement (INS(+)) or B-27 supplement (INS(-)) (Thermo Fisher Scientific) was further added to a basal medium (RPMI medium (RPMI 1640 medium (Thermo Fisher Scientific))) containing 100 ng/mL activin A and 3 µM CHIR99021 to prepare a differentiation-inducing medium.

The cells were seeded (day 0) and cultured according to the schedule described in Table 1 using the differentiation-inducing medium containing insulin or containing no insulin to induce their differentiation into definitive endoderm cells.

**[Table 1]**

| Presence or absence of insulin in differentiation-inducing medium and culture schedule | | | | |
|---|---|---|---|---|
| | day 0 | day 1 | day 2 | day 3 |
| Example | INS (+) | INS (+) | INS (-) | INS (-) |
| Comparative Example | INS (+) | INS (+) | INS (+) | INS (+) |

### (2) Results

In the case of first performing culture (day 0 to day 1) in a differentiation-inducing medium containing insulin and subsequently performing culture (day 2 to day 3) in a differentiation-inducing medium containing no insulin (Example), it was confirmed that the percentage of SOX2+ cells (indicating remaining undifferentiated cells or cells of other lineages) was decreased as compared with the case of culture only in a differentiation-inducing medium containing insulin (Comparative Example) (Ff-I01s04 line: Comparative Example: 2.1%, Example: 0.8%; Ff-I14s03 line: Comparative Example: 2.6%, Example: 0.8%) (Figure 1).

From these results, it was confirmed that in the induction of differentiation of pluripotent stem cells into definitive endoderm cells, the differentiation-inducing medium is changed from INS(+) medium to INS(-) medium, whereby the percentage of SOX2+ cells can be decreased and a cell population containing definitive endoderm cells with high purity can be produced.

### [II] Induction of differentiation into definitive endoderm cells, posterior foregut cells, and pancreatic progenitor cells involving change from INS(+) medium to INS(-) medium

### (1) Method

In the culture method described in the above section "I", an induced pluripotent cell line Ff-MH15s02 was used as pluripotent stem cells, and RPMI medium or DMEM medium (DMEM, high glucose, GlutaMAX(TM), pyruvate (Thermo Fisher Scientific)) was used as a basal medium. In the case of using RPMI medium as the base medium, the cells were cultured from day 0 through day 3 in a differentiation-inducing medium containing insulin. In the case of using DMEM medium as the base medium, the cells were first (day 0) cultured in a differentiation-inducing medium containing insulin and subsequently (day 1 to day 3) cultured in a differentiation-inducing medium containing no insulin. The pluripotent stem cells were seeded in an amount of 200 × 10⁴ cells/well (about 210,000 cells/cm²) or 250 × 10⁴ cells/well (about 260,000 cells/cm²). The induction of differentiation into definitive endoderm cells was performed by the same approach as in "Example" described above except for the procedures described here.

The obtained definitive endoderm cells were further induced to differentiate into posterior foregut cells and pancreatic progenitor cells.

### (2) Results

In the case of using DMEM medium as a basal medium and changing culture medium from INS(+) medium to INS(-) medium, it was confirmed that the percentage of SOX2+ cells was decreased at both the numbers of seeded cells, as compared with the case of culture using RPMI medium and involving insulin (DMEM medium: 4.8%, 3.1%; RPMI medium: 11.7%, 5.1%) (Figure 2). As a result of further inducing the differentiation of the obtained definitive endoderm cells into posterior foregut cells, the percentage of PDX1+ cells exceeded 90%. Also, as a result of inducing differentiation into pancreatic progenitor cells, the percentage of PDX1+/NKX6.1+ cells exceeded 80%. Thus, it was confirmed that the differentiation can be efficiently induced, as compared with culture in a differentiation-inducing medium containing insulin from day 0 through day 3(Figure 2).

From these results, it was confirmed that in the induction of differentiation of pluripotent stem cells into definitive endoderm cells, DMEM medium is used as a basal medium, and culture medium is changed from INS(+) medium to INS(-) medium, whereby the percentage of SOX2+ cells can be decreased; and the obtained definitive endoderm cells is further induced to differentiate, whereby cells including high purity of posterior foregut cells and pancreatic progenitor cells respectively can be produced. Although the Ff-MH15s02 line is a cell line that does not have high differentiation efficiency in the approach of the previous report (Stem Cell Research (2015) 14, 185-197), the approach of the present invention was confirmed to be able to induce the differentiation of even such a cell line with high efficiency.

### [III] Induction of differentiation into definitive endoderm cells using only INS(-) medium

### (1) Method

In the culture method described in the above section "II", an Ff-I01s04 line was used as pluripotent stem cells. In the case of using any base medium, the cells were cultured only in a differentiation-inducing medium supplemented with B-27 supplement (INS(-)), and the number of seeded cells was 70 × 10⁴ cells/well (about 70,000 cells/cm²). The induction of differentiation into definitive endoderm cells was performed by the same approach as described above except for the procedures described here (i.e., culture was performed only in a differentiation-inducing medium containing no insulin)

The obtained definitive endoderm cells were further induced to differentiate into posterior foregut cells.

### (2) Results

In the case of using RPMI medium as a basal medium, the percentage of obtained SOX2+ cells was 0.6% whereas the percentage of PDX1+ cells exhibited a value as low as 17.7%. The number of cells obtained after culture (on day 3) was decreased to 10 × 10⁴ cells/well (about 10,000 cells/cm²) .

In the case of using DMEM medium as a basal medium, the percentage of obtained SOX2+ cells exhibited a value as high as 6.7%, and the percentage of PDX1+ cells was 67.9%. The number of cells obtained after culture (on day 3) was 129 × 10⁴ cells/well (about 130,000 cells/cm²) .

From these results, it was confirmed that the induction of differentiation into definitive endoderm cells using only a differentiation-inducing medium containing no insulin may suppress increase in the number of cells and fail to obtain a sufficient number of differentiated cells, or elevates the rate of coexisting SOX2-positive (SOX2+) cells indicating remaining pluripotent stem cells or cells of other lineages, though the results differ depending on the medium.

### [IV] Induction of differentiation into definitive endoderm cells (three-dimensional culture) involving change from INS(+) medium to INS(-) medium

The method for inducing differentiation into definitive endoderm cells involving change from INS(+) medium to INS(-) medium, the effect of which was confirmed above, was confirmed to be also effective for a three-dimensional culture method using a bioreactor.

### (1) Method

An induced pluripotent cell line Ff-I14s04 was used as pluripotent stem cells.

The cells were seeded in an amount of 100,000 cells/mL to 10 mL of a medium for regenerative medicine (StemFit(R) (Ajinomoto Healthy Supply Co., Inc.)) supplemented with Y-27632 (10 µM), and cultured for 24 hours at a rotational speed of 70 rpm in a 30 mL reactor. Then, 20 mL of StemFit(R) was further added thereto, and the cells were further cultured for 48 hours.

Subsequently, a cell mass obtained after the culture was cultured for 24 hours at a rotational speed of 40 rpm in a 30 mL reactor containing a differentiation-inducing medium (30 mL) obtained by further adding B-27 supplement (INS(+)) to RPMI medium or DMEM medium containing 100 ng/mL activin A and 3 µM CHIR99021 (day 0).

Subsequently, a cell mass obtained after the culture was cultured for 48 hours at a rotational speed of 40 rpm in a 30 mL reactor containing RPMI medium containing 100 ng/mL activin A and B-27 supplement (INS(+)) or DMEM medium containing 100 ng/mL activin A and B-27 supplement (INS(-)) (30 mL each medium) (days 1 to 2) to obtain definitive endoderm cells.

For the obtained definitive endoderm cells, the differentiation-inducing medium was replaced with a fresh one, and the induction of differentiation was further performed by three-dimensional culture.

### (2) Results

As for the induction of differentiation of pluripotent stem cells into definitive endoderm cells by three-dimensional culture, in the case of performing culture using only RPMI medium containing insulin, the percentage of SOX2+ cells in an obtained cell mass exhibited 6.5% which was a high value as compared with that (0.4%) of a cell mass obtained by culture first using DMEM medium containing insulin and then using DMEM medium containing no insulin (Figure 3).

In the case of inducing the differentiation of the cell mass obtained by culture using only RPMI medium containing insulin into posterior foregut cells, an obtained cell mass had a percentage PDX1+ cells as small as about 20%, and decrease in the number of cells by the disruption of the cell mass was found (Figure 4). On the other hand, in the case of inducing the differentiation of the cell mass obtained by culture first using DMEM medium containing insulin and then using DMEM medium containing no insulin into posterior foregut cells, an obtained cell mass had a percentage of PDX1+ cells as high as more than 90% (Figure 3), and decrease in the number of cells was not found (Figure 4).

These results demonstrated that the method for inducing differentiation into definitive endoderm cells involving change from INS(+) medium to INS(-) medium is also effective for a three-dimensional culture method using a bioreactor and is capable of producing definitive endoderm cells or subsequent differentiated cells from pluripotent stem cells in a large amount at an industrial scale.

## Claims

1. A method for producing definitive endoderm cells from pluripotent stem cells, comprising
subjecting pluripotent stem cells to first culture in a differentiation-inducing medium in which insulin acts and subsequently to second culture in a differentiation-inducing medium in which insulin does not act.

2. The method according to claim **1,** wherein
the first culture is performed in a differentiation-inducing medium comprising insulin, and
the second culture is performed in a differentiation-inducing medium comprising no insulin.

3. The method according to claim **1,** wherein
the first culture is performed in a differentiation-inducing medium comprising insulin and comprising no insulin signaling inhibitor, and
the second culture is performed in a differentiation-inducing medium comprising insulin and an insulin signaling inhibitor.

4. The method according to any one of claims 1 to **3,** wherein the differentiation-inducing media in which the first culture and the second culture are performed further comprise pyruvate.

5. The method according to any one of claims 1 to **4,** wherein the differentiation-inducing media in which the first culture and the second culture are performed further comprise L-alanyl L-glutamine.

6. The method according to any one of claims 1 to **5,** wherein the differentiation-inducing media in which the first culture and the second culture are performed further comprise 15 mM or more glucose.

7. The method according to any one of claims 1 to **6,** comprising performing the first culture for 6 hours to 48 hours.

8. The method according to any one of claims 1 to **7,** comprising performing the second culture for at least 6 hours.

9. The method according to any one of claims 1 to **8,** wherein the method is carried out in a three-dimensional culture system.

10. The method according to any one of claims 1 to **8,** wherein the method is carried out in a two-dimensional culture system.

11. The method according to any one of claims 1 to 10, wherein the differentiation-inducing medium is based on DMEM (Dulbecco's modified Eagle medium).

12. The method according to any one of claims 1 to 11, wherein the differentiation-inducing medium for the first culture comprises a ROCK inhibitor and/or a GSK3β inhibitor.

13. A method for producing insulin-producing cells, comprising a step of further inducing the differentiation of definitive endoderm cells produced by subjecting pluripotent stem cells to first culture in a differentiation-inducing medium in which insulin acts and subsequently to second culture in a differentiation-inducing medium in which insulin does not act.

14. A method for producing insulin-producing cells, comprising a step of further inducing the differentiation of definitive endoderm cells produced by subjecting pluripotent stem cells to first culture in a differentiation-inducing medium comprising insulin and subsequently to second culture in a differentiation-inducing medium comprising no insulin.

15. A method for producing insulin-producing cells, comprising a step of further inducing the differentiation of definitive endoderm cells produced by subjecting pluripotent stem cells to first culture in a differentiation-inducing medium comprising insulin and comprising no insulin signaling inhibitor and subsequently to second culture in a differentiation-inducing medium comprising insulin and an insulin signaling inhibitor.

## Patentansprüche

1. Verfahren zur Herstellung definitiver Endodermzellen aus pluripotenten Stammzellen, umfassend
- Unterwerfen pluripotenter Stammzellen einer ersten Kultur in einem differenzierungs-induzierenden Medium, in dem Insulin wirkt, und anschließend einer zweiten Kultur in einem differenzierungsinduzierenden Medium, in dem Insulin nicht wirkt.

2. Verfahren nach Anspruch 1, wobei die erste Kultur in einem differenzierungsinduzierenden Medium durchgeführt wird, das Insulin enthält, und die zweite Kultur in einem differenzierungsinduzierenden Medium durchgeführt wird, das kein Insulin enthält.

3. Verfahren nach Anspruch 1, wobei
- die erste Kultur in einem differenzierungsinduzierenden Medium durchgeführt wird, das Insulin und keinen Insulinsignalisierungsinhibitor enthält, und
- die zweite Kultur in einem differenzierungsinduzierenden Medium durchgeführt wird, das Insulin und einen Insulinsignalisierungsinhibitor enthält.

4. Verfahren nach einem der Ansprüche 1 bis 3, wobei die differenzierungsinduzierenden Medien, in denen die erste Kultur und die zweite Kultur durchgeführt werden, ferner Pyruvat enthalten.

5. Verfahren nach einem der Ansprüche 1 bis 4, wobei die Differenzierungs-induzierenden Medien, in denen die erste Kultur und die zweite Kultur durchgeführt werden, ferner L-Alanyl-L-Glutamin umfassen.

6. Verfahren nach einem der Ansprüche 1 bis 5, wobei die differenzierungsinduzierenden Medien, in denen die erste Kultur und die zweite Kultur durchgeführt werden, ferner 15 mol/l oder mehr Glucose umfassen.

7. Verfahren nach einem der Ansprüche 1 bis 6, umfassend das Durchführen der ersten Kultur 6 Stunden bis 48 Stunden lang.

8. Verfahren nach einem der Ansprüche 1 bis 7, umfassend das Durchführen der zweiten Kultur mindestens 6 Stunden lang.

9. Verfahren nach einem der Ansprüche 1 bis 8, wobei das Verfahren in einem dreidimensionalen Kultursystem durchgeführt wird.

10. Verfahren nach einem der Ansprüche 1 bis 8, wobei das Verfahren in einem zweidimensionalen Kultursystem durchgeführt wird.

11. Verfahren nach einem der Ansprüche 1 bis 10, **dadurch gekennzeichnet, dass** das differenzierungsinduzierende Medium auf DMEM (Dulbecco's modified Eagle medium) basiert.

12. Verfahren nach einem der Ansprüche 1 bis 11, **dadurch gekennzeichnet, dass** das differenzierungsinduzierende Medium für die erste Kultur einen ROCK-Inhibitor und/oder einen GSK3ß-Inhibitor enthält.

13. Verfahren zur Herstellung von insulinproduzierenden Zellen, umfassend einen Schritt des weiteren Induzierens der Differenzierung von definitiven Endodermzellen, die hergestellt wurden, indem pluripotente Stammzellen einer ersten Kultur in einem differenzierungsinduzierenden Medium, in dem Insulin wirkt, und anschließend einer zweiten Kultur in einem differenzierungsinduzierenden Medium, in dem Insulin nicht wirkt, unterzogen wurden.

14. Verfahren zur Herstellung von insulinproduzierenden Zellen, umfassend einen Schritt des weiteren Induzierens der Differenzierung von definitiven Endodermzellen, die dadurch hergestellt wurden, dass pluripotente Stammzellen einer ersten Kultur in einem differenzierungsinduzierenden Medium, das Insulin enthält, und anschließend einer zweiten Kultur in einem differenzierungsinduzierenden Medium, das kein Insulin enthält, unterzogen wurden.

15. Verfahren zur Herstellung von Insulin-produzierenden Zellen, umfassend einen Schritt des weiteren Induzierens der Differenzierung von definitiven Endodermzellen, die hergestellt wurden, indem pluripotente Steinzellen einer ersten Kultur in einem differenzierungsinduzierenden Medium, das Insulin und keinen Insulinsignalisierungsinhibitor enthält, und anschließend einer zweiten Kultur in einem Differenzierungs-induzierenden Medium, das Insulin und einen Insulinsignalisierungsinhibitor enthält, unterzogen wurden.

## Revendications

1. Un procédé de production de cellules définitives de l'endoderme à partir de cellules souches pluripotentes, comprenant soumettre des cellules souches pluripotentes à une première culture dans un milieu induisant une différenciation, dans lequel agit de l'insuline, et ensuite à une deuxième culture dans un milieu induisant une différenciation dans lequel de l'insuline n'agit pas.

2. Le procédé suivant la revendication **1,** dans lequel
la première culture est effectuée dans un milieu induisant une différenciation comprenant de l'insuline et
la deuxième culture est effectuée dans un milieu induisant une différenciation ne comprenant pas d'insuline.

3. Le procédé suivant la revendication **1,** dans lequel
la première culture est effectuée dans un milieu induisant une différenciation comprenant de l'insuline et ne comprenant pas d'inhibiteur signalant de l'insuline et
la deuxième culture est effectuée dans un milieu induisant une différenciation comprenant de l'insuline et un inhibiteur signalant de l'insuline.

4. Le procédé suivant l'une quelconque des revendications 1 à **3,**
dans lequel les milieux induisant une différenciation, dans lesquels la première culture et la deuxième culture sont effectuées, comprennent en outre un pyruvate.

5. Le procédé suivant l'une quelconque des revendications 1 à 4,
dans lequel les milieux induisant une différenciation, dans lesquels la première culture et la deuxième culture sont effectuées, comprennent en outre de la L-alanyl L-glutamine.

6. Le procédé suivant l'une quelconque des revendications 1 à 5,
dans lequel les milieux induisant une différenciation dans lesquels la première culture et la deuxième culture sont effectuées, comprennent en outre 15 mM ou plus de glucose.

7. Le procédé suivant l'une quelconque des revendications 1 à 6,
comprenant effectuer la première culture pendant 6 heures à 48 heures.

8. Le procédé suivant l'une quelconque des revendications 1 à 7,
comprenant effectuer la deuxième culture pendant au moins 6 heures.

9. Le procédé suivant l'une quelconque des revendications 1 à 8,
dans lequel on effectue le procédé dans un système de culture à trois dimensions.

10. Le procédé suivant l'une quelconque des revendications 1 à 8,
dans lequel on effectue le procédé dans une système de culture à deux dimensions.

11. Le procédé suivant l'une quelconque des revendications 1 à 10,
dans lequel le milieu induisant une différenciation est à base de DMEM (milieu eagle modifié de Dulbecco).

12. Le procédé suivant l'une quelconque des revendications 1 à 11,
dans lequel le milieu induisant une différenciation pour la première culture comprend un inhibiteur ROCK et/ou un inhibiteur GSK3β.

13. Un procédé de production de cellules produisant de l'insuline,
comprenant un stade d'induction en outre de la différenciation de cellules définitives d'endoderme produites en soumettant des cellules souches pluripotentes à une première culture dans un milieu induisant une différenciation, dans lequel agit de l'insuline, et ensuite une deuxième culture dans un milieu induisant une différenciation, dans lequel de l'insuline n'agit pas.

14. Un procédé de production de cellules produisant de l'insuline,
comprenant un stade dans lequel on induit en outre la différenciation de cellules définitives d'endoderme produites en soumettant des cellules souches pluripotentes à une première culture dans un milieu induisant une différenciation comprenant de l'insuline et ensuite à une deuxième culture dans un milieu induisant une différenciation ne comprenant pas d'inuline.

15. Un procédé de production de cellules de production d'insuline,
comprenant un stade dans lequel on induit en outre la différenciation de cellules définitives d'endoderme produites en soumettant des cellules souches pluripotentes à une première culture dans un milieu induisant une différenciation comprenant de l'insuline et ne comprenant pas d'inhibiteur signalant de l'insuline et ensuite à une deuxième culture dans un milieu induisant une différenciation comprenant de l'insuline et un inhibiteur signalant de l'insuline.
